# EUROPÄISCHE PATENTANMELDUNG

(11) **EP 0 559 932 A1**
(43) Veröffentlichungstag der Anmeldung: **15.09.1993**
(21) Anmeldenummer: 92104097.8
(22) Anmeldetag: 10.03.1992
(51) Int. Cl.: A61N 1/05, A61N 1/39

(54) **Implantierbare Anordnung zum Defibrillieren oder Kardiovertieren eines Herzens**

(71) Anmelder: Pacesetter AB, S-171 95 Solna (SE)
(72) Erfinder: Alm, Malin, S-171 49 Solna (SE); Hirschberg, Jakub, S-183 44 Täby (SE); Peterson, Lars Olof, S-161 37 Bromma (SE); Stegfeldt, Olof, S-138 00 Älta (SE); Bowald, Staffan, S-749 10 Almunge (SE)
(74) Vertreter: Lettström, Richard Wilhelm

(57) **Zusammenfassung**

Eine Anordnung zum Defibrillieren eines Herzens weist eine erste Elektrode (18) in der vena cava inferior (17), eine zweite Elektrode (13) im rechten Ventrikel (9) und eine Flächenelektrode (20) außerhalb des Herzens auf.

## Beschreibung

Die Erfindung betrifft eine implantierbare Anordnung zum Defibrillieren oder Kardiovertieren eines Herzens mit impulsgenerierenden Mitteln und einer an diesen angeschlossenen Elektrodenanordnung bestehend aus zwei intravaskulären Elektroden, von denen eine über einen Katheter im rechten Ventrikel des Herzens angeordnet ist, und mit einer dritten Flächenelektrode außerhalb des Herzens im Bereich des linken Ventrikels, wobei die impulsgenerierenden Mittel zur gleichzeitigen Impulsbeaufschlagung aller drei Elektroden ausgebildet sind.

Bei einer derartigen, aus der US-A- 4 662 377 bekannten Anordnung sind an einem in die rechte Herzhälfte einzuführenden Katheter zwei Elektroden derart voneinander beabstandet angeordnet, daß die eine Elektrode im rechten Ventrikel des Herzens und die andere Elektrode in der vena cava superior zu liegen kommt. Außerhalb des Herzens ist eine dem linken Ventrikel gegenüberliegende Flächenelektrode subkutan angeordnet. Die Flächenelektrode ist mit der Elektrode in der vena cava superior verbunden und an einem Ausgangsanschluß eines implantierbaren Defibrillators angeschlossen, dessen anderer Ausgangsanschluß mit der Elektrode im Ventrikel verbunden ist, so daß im Fall der Defibrillierung des Herzens der Stromstoß in einen ersten Teilstrom zwischen der Elektrode im Ventrikel und der Elektrode in der vena ava superior und einen zweiten Teilstrom zwischen der Elektrode im Ventrikel und der Flächenelektrode aufgeteilt wird.

Aus der US-A- 4 708 145 ist eine weitere Anordnung zum Defibrillieren oder Kardiovertieren eines Herzens bekannt, die ebenfalls einen Katheter mit einer Elektrode im rechten Ventrikel und einer anderen Elektrode in der vena cava superior aufweist und bei der eine Flächenelektrode vorgesehen ist, die sowohl subkutan als auch epikardiell oder in der Nähe des Zwerchfells angeordnet werden kann. Die Abgabe von Defibrillierungsimpulsen erfogt sequentiell zwischen der Elektrode in der vena cava superior und der Elektrode im Ventrikel einerseits und der Flächenelektrode und der Elektrode im Ventrikel andererseits, so daß jeweils nur zwei Elektroden gleichzeitig bei der Impulsabgabe beteiligt sind und daher eine echte Verteilung der Stromdichte auf unterschiedliche Zonen des Herzmuskels nicht erfolgt. Anstelle in der vena cava superior kann die entsprechende Elektrode auch in der vena cava inferior angeordnet sein. Es ist ferner eine Elektrodenanordnung angegeben, bei der ausschließlich Fächenelektroden auf dem Herzen angeordnet sind.

Aus der EP-A-0 373 953 ist eine Anordnung zum Defibrillieren eines Herzens bekannt, bei der im Falle der Verwendung von drei Elektroden eine Elektrode über einen Katheter im rechten Ventrikel, eine weitere Elektrode über einen weiteren Katheter in der vena cordis magna und eine dritte Flächenelektrode subkutan dem linken Ventrikel gegenüberliegend angeordnet ist. Die Abgabe von Defibrillierungsimpulsen erfolgt entweder zwischen einer der genannten Elektroden und den beiden übrigen Elektroden, die dazu miteinander verbunden sind, oder es ist vorgesehen, daß die Elektroden paarweise nacheinander mit einem Defibrillierungsimpuls beaufschlagt werden.

Schließlich ist aus der DE-A-39 19 498 eine implantierbare Anordnung zum Defibrillieren oder Kardiovertieren eines Herzens bekannt, bei der eine von mehreren Elektroden im rechten Ventrikel des Herzens angeordnet ist und die übrigen Elektroden als Flächenelektroden außen am Herzen oder subkutan plaziert sind. Die Defibrillation erfolgt zwischen der Elektrode im Ventrikel und den miteinander verbundenen äußeren Elektroden, wobei sich die elektrische Stromdichte entsprechend der Anordnung der Elektroden verteilt und dabei vorzugsweise die dicksten Zonen des Hersmuskels durchdringt. Soll die Defibrillierungsenergie optimal ausgenutzt werden, so ist es erforderlich, daß die äußeren Elektroden direkt auf dem Herzen appliziert werden, wozu allerdings der Brustkorb geöffnet werden muß.

Der Erfindung liegt die Augabe zugrunde, eine Anordnung zum Defibrillieren eines Herzens anzugeben, mit der über eine entsprechende Verteilung der Stromdichte im Herzmuskel ein möglichst geringer Wert für die zur Defibrillierung des Herzens minimal erforderliche Defibrillierungsenergie erreicht wird.

Gemäß der Erfindung wird die Aufgabe dadurch gelöst, daß bei der Anordnung der eingangs angegebenen Art die andere intravaskuläre Elektrode im Bereich der vena cava inferior angeordnet ist. Mit einer solchen Anordnung wurden in Versuchen Werte für die Defibrillierungschwelle, also die minimal erforderliche Defibrillierungsenergie erreicht, die nicht größer, teilweise sogar kleiner waren, als bei Vergleichsanordnungen mit epikardiellen Elektoden. Im Unterschied zu diesen Vergleichsanordnungen war jedoch die Flächenelektrode subkutan angeordnet, so daß eine Öffnung des Brustkorbes nicht erforderlich war. Zwar ist aus der oben bereits genannten US-A-4 708 145 die Verwendung einer Elektrode in der vena cava inferior anstelle der vena cava superior bekannt, jedoch erfolgt dort die Abgabe der Defibrillierungsimpulse zu gleichen Zeitpunkten nur zwischen jeweils zwei Elektroden, so daß nicht die Verteilung der Stromdichte über den Herzmuskel erzielt wird, wie dies beim Gegenstand der Erfindung der Fall ist.

Für den Fall, daß die intravaskulären Elektroden über die vena cava inferior in das intravaskuläre System eingeführt werden sollen, sind vorzugsweise die Elektrode in dem Ventrikel und die Elektrode in der vena cava inferior an demselben Katheter ausgebildet. Dadurch wird erreicht, daß der Abstand zwischen den beiden intravaskulären Elektroden genau definiert ist und daß sich beide Elektroden gleichzeitig an den für sie vorgesehenen Stellen positionieren lassen.

Für den Fall, daß die Positionierung der Elektrodenanordnung von der vena cava superior her erfolgen soll, ist vorzugsweise die Elektode in der vena cava inferior an einem eigenen zweiten Katheter ausgebildet. Um dabei eine stabile Lage des zweiten Katheters in seiner implantierten Endstellung zu erreichen, weist der zweite Katheter mit der Elektrode in der vena cava inferior in vorteilhafter Weise an seinem distalen Endbereich Mittel zur Fixierung der Elektrode in der vena cava inferior auf. Diese Mittel können beispielsweise in Form von Spreizelementen oder duch eine schraubenfederartige Ausbildung der Elektrode in der vena cava inferior realisiert werden, wodurch das distale Ende des Katheters in der Vene festgeklemmt wird. Eine weitere Möglichkeit besteht darin, daß das distale Ende mit Hilfe von Haken oder schraubenlinienförmigen Fortsätzen in der Venenwand verankerbar ist.

Entsprechend einer bevorzugten Ausbildung der erfindungsgemäßen Anordnung weist der zweite Katheter mit der Elektrode in der vena cava inferior Mittel zu seiner Fixierung im Bereich des rechten Atriums auf. Dadurch wird verhindert, daß der zweite Katheter in dem relativ großräumigen Venenbereichen seine Lage verändern kann und unter Umständen in das Innere des Herzens geraten kann. Die Mittel zum Fixieren des zweiten Katheters im Bereich des rechten Atriums bestehen dabei vorzugsweise aus einer von einem geradlinigen Verlauf abweichenden Verformung des Katheters oder aus seitlich vom Katheter abstehenden Spreizelementen. Dabei ist vorgesehen, daß diese Fixierungsmittel erst dann aktiviert werden, wenn der Katheter in seiner endgültigen Lage positioniert worden ist. Die von dem geradlinigen Verlauf abweichende Verformung des Katheters kann dabei in der Weise bewerkstelligt werden, daß der Katheter an der entsprechenden Stelle vorgebeugt ist und nach Entfernen eines im Inneren des Katheters geführten Steuerdrahtes die vorgesehene Verformung annimmt. Eine weitere Möglichkeit zur Verformung des Katheters besteht darin, daß dieser Elemente aus einer Metallegierung enthält, die beim Erreichen eines vorgegebenen, hier vorzugsweise der Körpertemperatur entsprechenden Temperaturniveaus eine vorgegebene Form annehmen.

Entsprechend einer vorteilhaften Weiterbildung der erfinungsgemäßen Anordnung, die eine Mehrfachausnutzung des Katheters ermöglicht, ragen die Mittel zum Fixieren des zweiten Katheters in den Bereich des rechten Atriums hinein und weisen dort eine atrielle Elektrode auf, die über eine innerhalb des Katheters geführte Elektrodenleitung mit einer Detektor- und/oder Herzschrittmacherschaltung verbunden ist.

Im einfachsten Fall sind jeweils zwei der Defibrillierungselektroden, vorzugsweise die Elektrode in der vena cava inferior und die Flächenelektrode, miteinander verbunden. Damit wird erreicht, daß sich im Fall der Defibrillierung des Herzens der Stromstoß in zwei Teilströme zwischen jeweils einer der beiden miteinander verbundenen Elektroden und der übrigen Elektrode aufteilt.

Bei einer besonders vorteilhaften Ausbildung der erfindungsgemäßen Anordnung sind die impulsgenerierenden Mittel zur gleichzeitigen Beaufschlagung der drei Elektroden mit jeweils unterschiedlichen Spannungen ausgebildet. Dadurch wird erreicht, daß die Stromverteilung im Herzen nicht nur von der Anordnung der Elektroden abhängig ist, sondern zusätzlich über die unterschiedlichen elektrischen Spannungen an den Ausgängen der impulsgenerierenden Mittel eingestellt werden kann.

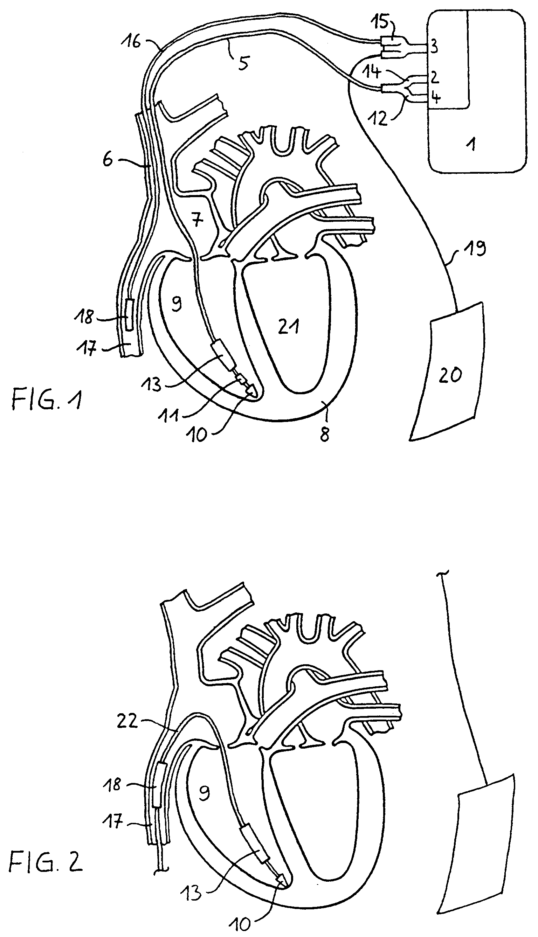

Zur Erläuterung der Erfindung wird im Folgenden auf die Figuren der Zeichnung Bezug genommen, wobei die
Figuren 1 bis 6 unterschiedliche Ausführungsbeispiele der erfindungsgemäßen Anordnung zeigen.

Figur 1 zeigt einen implantierbaren Defibrillator/Kardiovertierer 1 mit zwei Ausgangsanschlüssen 2 und 3 zur Abgabe von Defibrillierungsimpulsen und einem weiteren bipolaren Anschluß 4 der mit einer hier nicht gezeigten Detektor- und Herzschrittmacherschaltung im Inneren des Defibrillators/Kardiovertierers 1 verbunden ist. An den Ausgangsanschlüssen 2 und 4 ist ein Katheter 5 angeschlossen, der durch die vena cava superior 6 und das rechte Atrium 7 des Herzens 8 hindurch in den rechten Ventrikel 9 geführt ist und dort an seinem distalen Ende eine Spitzenelektrode 10 und eine von dieser in proximaler Richtung beabstandete Ringelektrode 11 aufweist, die beide über hier nicht gezeigte Elektrodenleitungen im Inneren des Katheters 5 und eine erste Verzweigung 12 an seinem proximalen Ende mit dem bipolaren Anschluß 4 des Defibrillators/Kardiovertierers 1 verbunden sind. Der Katheter 5 weist ferner eine im rechten Ventrikel 9 angeordnete Defibrillierungselektrode 13 auf, die über eine ebenfalls hier nicht gezeigte Leitung im Inneren des Katheters 5 und eine zweite Verzweigung 14 an seinem proximalen Ende mit dem Ausgangsanschluß 2 des Defibrillators/Kardiovertierers 1 verbunden ist. In den zweiten Ausgangsanschluß 3 des Defibrillators/Kardiovertierers 1 ist ein Adapterstück 15 eingefügt, an dem zum einen eine über einen zweiten Katheter 16 in der vena cava inferior 17 positionierte zweite Defibrillierungselektrode 18 angeschlossen ist und zum anderen über eine isolierte Leitung 19 eine Flächenelektrode 20 angeschlossen ist, die im Bereich des linken Ventrikels 21 des Herzens 8 an diesem oder im Abstand zum Herzen 8 subkutan implantiert ist. Bei dem in Figur 1 gezeigten Ausführungsbeispiel erfolgt die Defibrillation des Herzens 8 zwischen der Elektrode 13 im rechten Ventrikel 9 einerseits und den miteinander verbundenen Elektroden 18 und 20 in der vena cava inferior bzw. außerhalb des Herzens 8 andererseits. Die dazu minimal erforderliche Defibrillierungsenergie erreicht Werte, die nicht größer, teilweise sogar kleiner sind, als bei Vergleichsanordnungen mit epikardiellen Elektroden.

Das in Figur 2 gezeigte Ausführungsbeispiel der erfindungsgemäßen Anordnung unterscheidet sich von dem zuvor beschriebenen Ausführungsbeispiel dadurch, daß die Defibrillierungselektrode 13 im rechten Ventrikel 9 und die Elektrode 18 in der vena cava inferior 17 an einem gemeinsamen Katheter 22 angeordnet sind, der über die vena cava inferior 17 hindurch in das intravaskuläre System eingeführt ist. Ferner dient bei dem in Figur 2 gezeigten Ausführungsbeispiel die Defibrillierungselektrode 13 im Ventrikel 9 als Gegenelektrode zur Spitzenelektrode 10, weswegen der Katheter 22 keine Ringelektrode (10, Figur 1) aufweist.

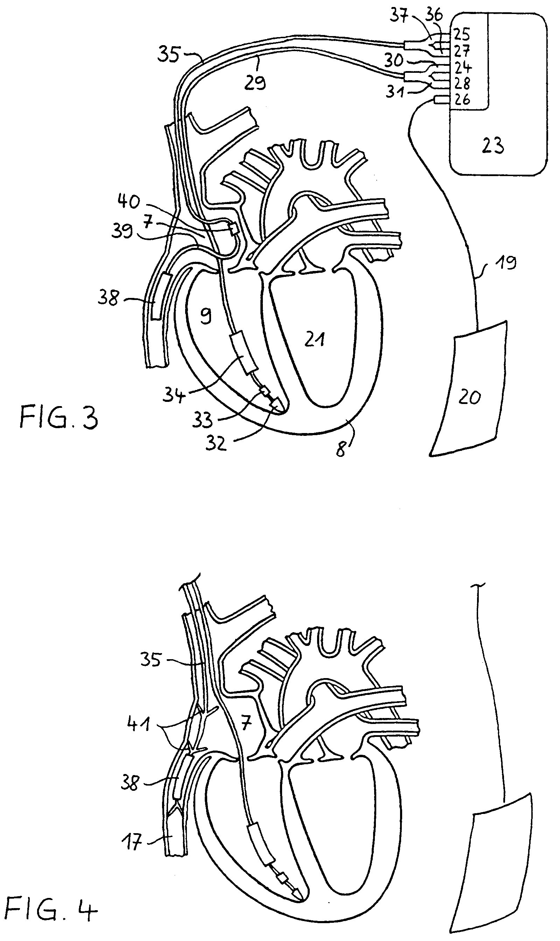

Figur 3 zeigt einen implantierbaren Defibrillator/Kardiovertierer 23 mit 3 Ausgangsanschlüssen 24, 25 und 26 zur Abgabe von Defibrillierungsimpulsen sowie mit einem weiteren unipolaren Anschluß 27 und einem bipolaren Anschluß 28, die beide mit einer hier nicht gezeigten Detektor- und Herzschrittmacherschaltung im Inneren des Defibrillators/Kardiovertierers 23 verbunden sind. Ein erster Katheter 29 ist an seinem proximalen Ende in zwei Anschlußstücke 30 und 31 verzweigt, die mit den Anschlüssen 24 und 28 des Defibrillators/Kardiovertierers 23 verbunden sind. Der Katheter 29 ist durch die vena cava superior 6 und das rechte Atrium 7 des Herzens 8 hindurch in den rechten Ventrikel 9 geführt und weist dort an seinem distalen Ende eine Spitzenelektrode 32 und eine von dieser in proximaler Richtung beabstandete Ringelektrode 33 auf, die beide über hier nicht gezeigte Elektrodenleitungen im Inneren des Katheters 29 und den bipolaren Anschluß 28 mit der Detektor- und Herzschrittmacherschaltung in dem Defibrillator/Kardiovertierer 23 verbunden sind. Der Katheter 29 weist weiterhin eine Defibrillierungselektrode 34 im Ventrikel 9 auf, die über eine hier ebenfalls nicht gezeigte Leitung im Inneren des Katheters 29 mit dem Anschluß 24 des Defibrillators/Kardiovertierers 23 verbunden ist. An den Anschlüssen 25 und 27 ist ein weiterer Katheter 35 angeschlossen, der dazu an seinem proximalen Ende ebenfalls in zwei Anschlußstücke 36 und 37 verzweigt ist. Der zweite Katheter 35 weist an seinem distalen Ende eine Defibrillierungselektrode 38 auf, die in der vena cava inferior 17 positioniert ist und über eine hier nicht gezeigte Leitung im Inneren des Katheters 35 mit dem Anschluß 25 des Defibrillators/Kardiovertierers 23 verbunden ist. Im Bereich zwischen der vena cava superior 6 und der vena cava inferior 17 weist der zweite Katheter 35 eine von dem geradlinigen Verlauf abweichende Verformung 39 auf. Die Verformung 39 des Katheters 35 reicht in das Atrium 7 des Herzens 8 hinein und weist dort eine atrielle Elektrode 40 auf, die über eine hier nicht gezeigte Elektrodenleitung innerhalb des Katheters 35 und den unipolaren Anschluß 27 mit der Detektor- und/oder Herzschrittmacherschaltung des Defibrillators/Kardiovertierers 23 verbunden ist. An dem dritten Defibrillierungsanschluß 26 ist über eine isolierte elektrische Leitung 19 eine Flächenelektrode 20 angeschlossen, die subkutan dem linken Ventrikel 21 des Herzens 8 gegenüberliegend angeordnet ist. Anstelle der atriellen Elektrode ist auch eine Meßsonde für Druck, Strömung, Temperatur oder Gassättigung des venösen Bluts denkbar. Zum Defibrillieren des Herzens 8 erzeugt der Defibrillator/Kardiovertierer zwischen seinen Ausgangsanschlüssen 24,25 und 26 unterschiedliche Spannungen wodurch eine Stromverteilung im Herzen 8 erreicht wird, die nicht nur von der Anordnung der Elektroden 20,34 und 38 abhängig ist, sondern zusätzlich über die unterschiedlichen Spannungen zwischen den Elektroden 20,34 und 38 einstellbar ist.

Figur 4 zeigt schließlich ein Ausführungsbeispiel der erfindungsgemäßen Anordnung, bei dem der zweite Katheter 35 im Bereich des Atriums 7 sowie im Bereich der Elektrode 38 in der vena cava inferior 17 Spreizelemente 41 zur Fixierung des Katheters 35 aufweist.

### Bezugszeichenliste

- 1: Defibrillator/Konvertierer
- 2,3: Ausgangsanschlüsse von 1
- 4: bipolarer Anschluß von 1
- 5: Katheter
- 6: vena cava superior
- 7: rechtes Atrium
- 8: Herz
- 9: rechter Ventrikel
- 10: Spitzenelektrode
- 11: Ringelektrode
- 12: erste Verzweigung von 5
- 13: Defibrillierungselektrode
- 14: zweite Verzweigung von 5
- 15: Adapterstück
- 16: zweiter Katheter
- 17: vena cava inferior
- 18: zweite Defibrillierungselektrode
- 19: Leitung
- 20: Flächenelektrode
- 21: linker Ventrikel
- 22: Katheter
- 23: Defibrillator/Kardiovertierer
- 24,25,26: Ausgangsanschlüsse von 23
- 27: unipolarer Anschluß von 23
- 28: bipolaarer Anschluß von 23
- 29: erster Katheter
- 30,31: Anschlußstücke von 29
- 32: Spitzenelektrode
- 33: Ringelektrode
- 34: Defibrillierungselektrode
- 35: weiterer Katheter
- 36,37: Anschlußstücke von 35
- 38: Defibrillierungselektrode
- 39: Verformung von 35
- 41: Spreizelemente

## Patentansprüche

1. Implantierbare Anordnung zum Defibrillieren oder Kardiovertieren eines Herzens (8) mit impulsgenerierenden Mitteln (1;23) und einer an diesen angeschlossenen Elektrodenanordnung bestehend aus zwei intravaskulären Elektroden (13,18; 34,38), von denen eine (13;34) an einem Katheter (5,22;29) im rechten Ventrikel (9) des Herzens (8) angeordnet ist, und mit einer dritten Flächenelektrode (20) außerhalb des Herzens (8) im Bereich des linken Ventrikels (21), wobei die impulsgenerierenden Mittel (1;23) zur gleichzeitigen Impulsbeaufschlagung aller drei Elektroden (13,18,20;34,38,20) ausgebildet sind, **dadurch gekennzeichnet**, daß die andere intravaskuläre Elektrode (18,38) im Bereich der vena cava inferior (17) angeordnet ist.

2. Anordnung nach Anspruch 1, **dadurch gekennzeichnet,** daß die Elektrode (13) im Ventrikel (9) und die Elektrode (18) in der vena cava inferior (17) an demselben Katheter (22) ausgebildet sind.

3. Anordnung nach Anspruch 1, **dadurch gekennzeichnet**, daß die Elektrode (18,38) in der vena cava inferior (17) an einem eigenen zweiten Katheter (16,35) ausgebildet ist.

4. Anordnung nach Anspruch 3, **dadurch gekennzeichnet**, daß der zweite Katheter (35) mit der Elektrode (38) in der vena cava inferior (17) an seinem distalen Endbereich Mittel (41) zur Fixierung der Elektrode (38) in der vena cava inferior (17) aufweist.

5. Anordnung nach Anspruch 3 oder 4, **dadurch gekennzeichnet**, daß der zweite Katheter (35) mit der Elektrode (38) in der vena cava inferior (17) Mittel (39,41) zu seiner Fixierung im Bereich des rechten Atriums (7) aufweist.

6. Anordnung nach Anspruch 5, **dadurch gekennzeichnet,** daß die Mittel zum Fixieren des zweiten Katheters (35) im Bereich des rechten Atriums (7) aus einer von einem geradlinigen Verlauf abweichenden Verformung (39) des Katheters (35) bestehen.

7. Anordnung nach Anspruch 5, **dadurch gekennzeichnet**, daß die Mittel zum Fixieren des zweiten Katheters (35) im Bereich des rechten Atriums (7) aus seitlich von dem Katheter (35) abstehenden Spreizelementen (41) bestehen.

8. Anordnung nach einem der Ansprüche 5 bis 7, **dadurch gekennzeichnet**, daß die Mittel (39) zum Fixieren des zweiten Katheters (35) in das rechte Atrium (7) hineinragen und dort eine atrielle Elektrode (40) aufweisen, die über eine innerhalb des Katheters (35) geführte Elektrodenleitung mit einer Detektor- und/oder Herzschrittmacherschaltung verbunden ist.

9. Anordnung nach einem der vorangehenden Anspruche, **dadurch gekennzeichnet**, daß jeweils zwei der Elektroden, vorzugsweise die Elektrode (18) in der vena cava inferior (17) und die Flächenelektrode (20) miteinander verbunden sind.

10. Anordnung nach einem der Ansprüche 1 bis 8, **dadurch gekennzeichnet**, daß die impulsgenerierenden Mittel (23) zur gleichzeitigen Beaufschlagung der drei Elektroden (13,18,20) mit jeweils unterschiedlichen Spannungen ausgebildet sind.
